# EUROPÄISCHE PATENTSCHRIFT

(11) **EP 2 570 143 B1**
(45) Veröffentlichungstag und Bekanntmachung des Hinweises auf die Patenterteilung: **15.01.2014**
(21) Anmeldenummer: 12180877.8
(22) Anmeldetag: 17.08.2012
(51) Int. Cl.: A61M 1/10, A61N 1/39

(54) **Implantierbares kardiales Therapiergerät**
Implantable cardiac therapy device
Appareil thérapeutique cardiaque implantable

(30) Priorität: 14.09.2011 US 201161534392 P
(43) Veröffentlichungstag der Anmeldung: 20.03.2013
(73) Patentinhaber: BIOTRONIK SE & Co. KG, 12359 Berlin (DE)
(72) Erfinder: Dörr, Thomas, 12437 Berlin (DE); Weiss, Ingo, 12435 Berlin (DE)
(74) Vertreter: Lindner-Vogt, Karin L.

(56) Entgegenhaltungen:
- WO-A1-2006/020942
- WO-A2-2006/122036
- WO-A2-2010/030904
- US-A1- 2003 074 144

## Beschreibung

Die Erfindung betrifft ein implantierbares kardiales Therapiegerät.

Bekannte implantierbare kardiale Therapiegeräte sind z.B. Herzunterstützungspumpen, die mit einem Ventrikel eines Herzens und einer zugehörigen Arterie (Pulmonalarterie bzw. Aorta) zu verbinden und ausgebildet sind, im Einsatzfall Blut aus einem jeweiligen Ventrikel unterstützend in eine jeweils zugehörige Arterie (Pulmonalarterie, Aorta) zu pumpen und so den jeweiligen Ventrikel zu entlasten.

Andere bekannte implantierbare kardiale Therapiegeräte sind Herzstimulatoren wie beispielsweise Herzschrittmacher oder Defibrillatoren/Kardioverter mit einer Defibrillationseinheit zur automatischen Defibrillation eines Herzkammerflimmerns.

Ein Gerät gemäß des Präambel von Anspruch 1 ist aus WO 2010030904 bekannt.

Es ist auch bekannt, einen Patienten mit mehreren solcher Geräte zu versorgen.

Erfindungsgemäß wird ein Kombinationstherapiegerät zur Steigerung der Defibrillationstherapieeffizienz bei Patienten mit sehr stark eingeschränkter ventrikulärer Pumpfunktion vorgeschlagen, das eine Kombination eines implantierbaren Kardioverters/Defibrillators (ICD) und einer ventrikulären Assistenzpumpe (ventricular assist device, VAD) - hier als Herzunterstützungspumpe bezeichnet - darstellt und eine Steuereinheit aufweist, die mit der Herzunterstützungspumpe und der Defibrillationseinheit steuernd verbunden und ausgebildet ist, im Falle eines Herzkammerflimmerns die Herzunterstützungspumpe und die Defibrillationseinheit derart koordiniert anzusteuern, dass die Herzunterstützungspumpe zunächst ihre Leistung steigert um im Einsatzfall zunächst eine Druckentlastung wenigstens eines unterstützen Ventrikels zu bewirken und die Defibrillationseinheit erst anschließend, wenn eine ventrikuläre Druckentlastung vorliegt, einen Defibrillationsschock abgibt.

Die Steuereinheit bildet somit eine Therapiekoordinationseinheit, die im Falle eines Herzkammerflimmerns zunächst die Leistung der Unterstützungspumpe derart anhebt, dass zunächst eine Druckentlastung des/der unterstützen Ventrikel stattfindet und danach erst eine elektrische Defibrillation ausgelöst wird, wenn die ventrikuläre Druckentlastung vorliegt.

Die Erfindung schließt die Erkenntnis ein, dass bei Patienten mit sehr stark eingeschränkter ventrikulärer Pumpfunktion ein deutlich erhöhtes Risiko eines ventrikulären Flimmerns (VF) besteht. Zugleich ist gerade bei diesen Patienten die Therapieeffizienz einer rein elektrischen Defibrillation eingeschränkt. Grund hierfür ist der Auslösemechanismus für VF in diesem Patienten: das spontan aufgetretene VF führt durch den venösen Rückstrom zu einer zusätzlichen Füllung des nicht mehr pumpenden Herzens und damit zu einer Steigerung der Wandspannung des Myokard. Diese überhöhte Wandspannung ist wiederum Ursache für ein intrinsisches Flimmern, d.h. ohne vorherige Reduktion der Wandspannung/Ventrikelfüllung bleibt eine elektrische Defibrillation wirkungslos.

Zusätzlich bedingt eine bei o.g. Patienten auftretende Myokardischämie ebenfalls eine Verschlechterung der Defibrillationsprognose.

Die genannten Patienten erfüllen häufig die Indikation für eine ventrikuläre Unterstützungspumpe (VAD), da bei diesen Patienten der Grad der Dekompensation derart fortgeschritten ist, dass eine ausreichende Sauerstoffversorgung von Körperkreislauf und Myokardperfusion nicht mehr gegeben ist. Diese VAD-Systeme sind derzeit als reine Pumpensysteme ausgelegt und sind nicht in der Lage, ein spontanes VF zu therapieren. Da die Pumpenfunktion auch währen eines VF einen Kreislauf aufrecht erhält, können diese Patienten extern defibrilliert werden, sofern schnell genug eine Notärztliche Versorgung gegeben ist.

Das erfindungsgemäße implantierbare kardiale Therapiegerät hat den Effekt, die Therapieeffizienz einer automatischen elektrischen Defibrillation bei Patienten mit sehr stark eingeschränkter Pumpfunktion signifikant zu steigern.

Vorzugsweise weist das implantierbare kardiale Therapiegerät einen Drucksensor z.B. als Bestandteil der Herzunterstützungspumpe auf, der mit der Steuereinheit verbunden ist. Die Steuereinheit kann dann so ausgebildet sein, dass sie die Steuerung der Druckentlastung vor Defibrillation auf Basis des Ausgangssignals des Drucksensors vornimmt.

Vorzugsweise ist die Defibrillationseinheit mit wenigstens einer zur Abgabe eines Defibrillationsschocks gestalteten Defibrillationsslektrode verbunden, die Bestandteil der Herzunterstützungspumpe oder eines Fluidanschlusses der Herzunterstützungspumpe ist. Die Defibrillationselektroden sind somit integraler Bestandteil der Unterstützungspumpe.

Zusätzlich kann das implantierbare kardiale Therapiegerät einen Sauerstoffsensor aufweisen, der mit der Steuereinheit verbunden ist und im Betrieb ein den Blutsauerstoffgehalt anzeigendes Ausgangssignal liefert. Die Steuereinheit ist dann vorzugsweise so ausgebildet, dass sie eine Defibrillationsschockabgabe zusätzlich auf Basis des Ausgangssignals des myokardialen Sauerstoffsensors steuert.

Außerdem kann das implantierbare kardiale Therapiegerät einen programmierbaren Zeitgeber aufweisen, der mit der Steuereinheit verbunden oder Bestandteil derselben ist. Die Steuereinheit ist dann vorzugsweise so ausgebildet, dass sie die Steuerung der Defibrillationsschockabgabe zusätzlich mit Hilfe des programmierbaren Zeitgebers so steuert, dass vor der Defibrillation eine definierte Laufzeit der Unterstützungspumpe mit gesteigerter Leitung sichergestellt ist.

Gemäß einer weiteren bevorzugten Ausführungsvariante umfasst das implantierbare kardiale Therapiegerät zusätzlich eine Stimulationseinheit zur Post-Schock-Stimulation, die mit der Steuereinheit verbunden und ausgebildet ist, gesteuert durch die Steuereinheit im Bedarfsfall elektrische Stimulationspulse zur Stimulation einer Herzkammerkontraktion zu liefern.

Auch kann das implantierbare kardiale Therapiegerät als Kombinationstherapiegerät einen Herzstimulator oder wenigstens eine durch die Steuereinheit gesteuerte Stimulationseinheit aufweisen, die ggf. in Kombination mit der Steuereinheit dazu ausgebildet ist, beide Ventrikel eines Herzens im Sinne einer kardialen Resynchronisationstherapie (CRT) zu stimulieren.

Vorzugsweise weist das implantierbare kardiale Therapiegerät einen Impedanzsensor zum Erfassen einer intrakardialen Impedanz und einen Herzstimulator oder wenigstens eine durch die Steuereinheit gesteuerte Stimulationseinheit auf, welche mit der Steuereinheit verbunden sind. Die Steuereinheit kann dann ausgebildet sein, eine Abgabe von Stimulationsimpulsen in Abhängigkeit eines eine intrakardiale Impedanz anzeigen Ausgangssignals des Impedanzsensors zu steuern. Insbesondere kann die Steuereinheit in Verbindung mit dem Impedanzsensor und der Stimulationseinheit dazu ausgebildet sein, die Stimulation auf Basis eines eine kardiale Kontraktilität anzeigenden Ausgangssignals des Impedanzsensors derart zu steuern, dass eine bedarfsgerechte Steigerung der Kontraktilität resultiert. Auf diese Weise ergibt sich ein Kombinationstherapiegerät mit einem CCM-Stimulator (Cardiac Contractility Modulation) zur bedarfsgerechten Steigerung der Kontraktilität.

Außerdem kann das implantierbare kardiale Therapiegerät Mittel zur Erfassung und Auswertung rhythmologischer Informationen aufweisen und die Steuereinheit dazu ausgebildet sein, die Herzunterstützungspumpe auf Basis rhythmologischer Informationen so zu steuern, dass die Herzunterstützungspumpe eine Herz bedarfsgerecht entlastet. Die Mittel zur Erfassung und Auswertung rhythmologischer Informationen umfassen vorzugsweise eine oder mehrere Wahrnehmungseinheiten, die mit der Steuereinheit verbunden sind, wobei die Mittel zur Erfassung und Auswertung rhythmologischer Informationen ausgebildet sind, ventrikuläre Arrhythmien wie Kammerflimmern und ventrikuläre Tachykardien zu erkennen und zu unterscheiden. Die Mittel zur Erfassung und Auswertung rhythmologischer Informationen können in diesem Fall beispielsweise durch eine entsprechend ausgebildete Steuereinheit und die daran angeschlossenen Wahrnehmungseinheiten realisiert sein. Die Erfassung und Auswertung rhythmologischer Informationen kann beispielsweise auf Basis ans sich bekannter Algorithmen oder Kriterien erfolgen wie z.B. der SMART Algorithmus, Morphologiekriterien etc..

Darüber hinaus ist jede Kombination von mono- und biventrikulären Herzunterstützungspumpen (VAD-Pumpen) mit bekannten Systemen zur kardialen Elektrotherapie denkbar und im Einzelfall nützlich.

Die Erfindung soll nun anhand von Ausführungsbeispielen mit Bezug auf die Figuren näher erläutert werden. Diese zeigen in:
- Figur 1:: eine Darstellung des klinischen Problems von Patienten mit stark ausgeprägter Herzinsuffizienz;
- Figur 2:: ein erfindungsgemäßes implantierbares kardiales Therapiegerät als Kombinationsgerät aus Herzunterstützungspumpe und Defibrillator;
- Figur 3:: ein Blockschaltbild des Kombinationstherapiegerätes aus Figur 2; und
- Figur 4:: ein beispielhaftes Ablaufdiagramm für die Steuerung der Kombinationstherapie.

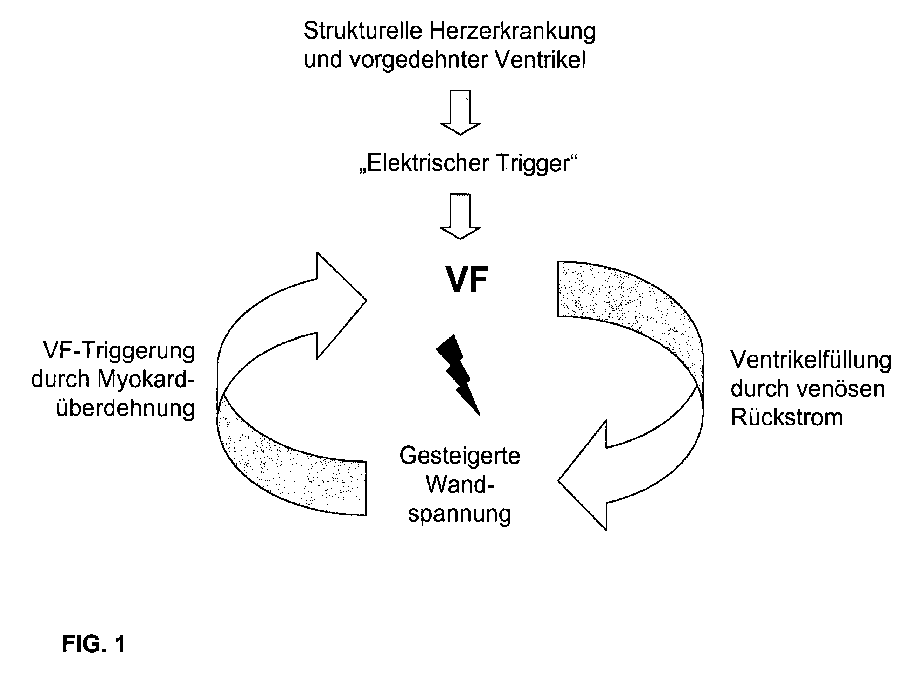

In der Figur 1 ist das klinische Problem der Patienten mit stark ausgeprägter Herzinsuffizienz dargestellt. Diese Patienten leiden unter einer ausgeprägten strukturellen Hererkrankung, einhergehend mit einem auf Grund überhöhter Druckverhältnisse stark vorgedehntem linken/rechten Ventrikel(n). Diese Bedingungen steigern deutlich die Vulnerabilität für einen elektrischen Trigger zum Auslösen eines spontanen ventrikulären Flimmerns. Aus diesem Grund werden diese Patienten auch mit einem implantierten Defibrillator (typischerweise in Kombination mit einem Resynchronisationsstimulator CRT-D) versorgt.

Allerdings ist die Therapieeffizienz der elektrischen Defibrillation bei diesen Patienten limitiert, da bei einem ventrikulären Flimmern keine Pumpfunktion des Herzens mehr besteht, der venöse Rückstrom jedoch das Blutvolumen im Herzen deutlich steigert. Dadurch erfolgt eine gesteigerte ventrikuläre Wandspannung, die ihrerseits einen weiteren Triggermechanismus für ein VF darstellt. Da die elektrische Defibrillation nur die elektrische Aktivität des VF "zurücksetzen" kann, nicht jedoch die Wandspannung lösen, verbleibt der Patient in einem therapierfraktärem ventrikulären Kammerflimmern.

Aus diesem Grund wird häufig bei schwer herzinsuffizienten Patienten bei der CRT-D-Implantation auf die Defibrillationstestung verzichtet.

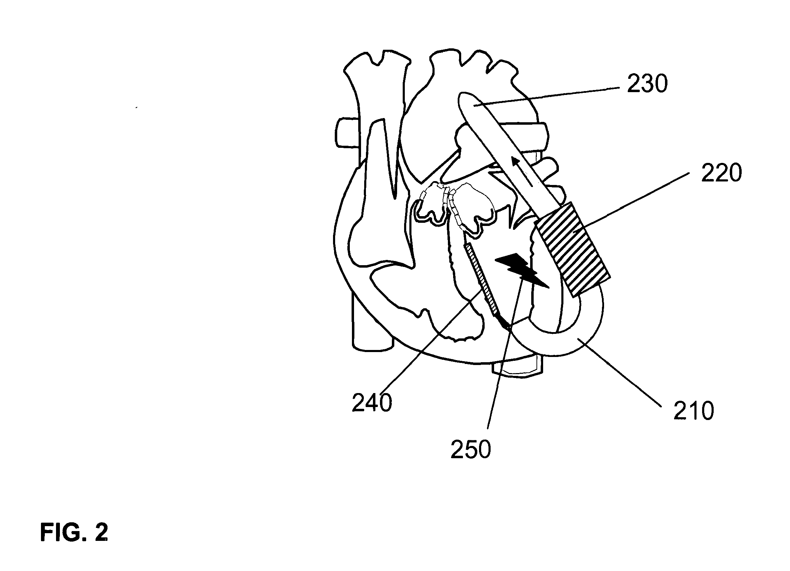

In der Figur 2 ist das erfindungsgemäße Gesamtsystem in einem Ausführungsbeispiel dargestellt. Zum Anschluss der Unterstützungspumpe (220) wir eine Zuleitung (210) in den linken Ventrikel des Patienten implantiert. Diese Zuleitung hat dabei die Aufgabe, einen Teil des Blutes vom linken Ventrikel zur Pumpe (220) fließen zu lassen. Die Unterstützungspumpe wiederum pumpt das Blutvolumen in die Aorta (230) und entlastet so den linken Ventrikel. Zusätzlich weist das erfindungsgemäße System eine Defibrillations- und Wahrnehmungselektrode (240) im linken Ventrikel auf. Da die Patienten durch die Assistenzpumpe ohnehin antikoaguliert werden, ist die Implantation einer linksventrikuläre Elektrode unkritisch. Als Gegenelektrode und Defibrillationsgenerator dient bei dieser Anordnung das Pumpengehäuse der Assistenzpumpe (220), d.h. der Defibrillationsschock (250) wird zwischen Pumpengehäuse (220) und linksventrikulärer Schockelektrode (240) abgegeben.

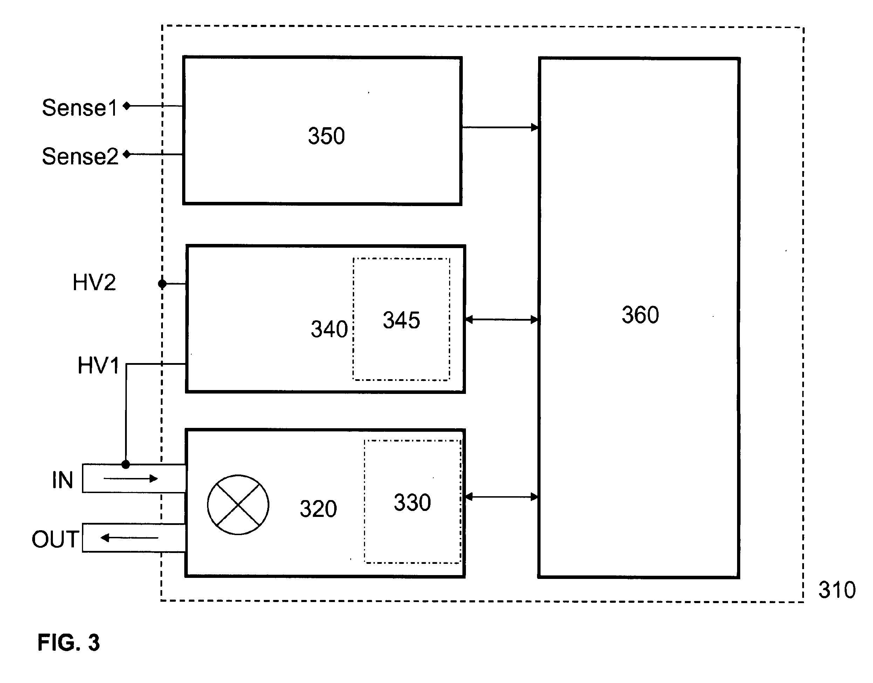

In der Figur 3 ist das Blockschaltbild des implantierbaren kardialen Therapiegerätes (310) in Form eines Kombinationstherapiegerätes dargestellt. Innerhalb des VAD-Pumpengehäuses (d.h. des Gehäuses der Herzunterstützungspumpe) befindet sich die eigentliche Pumpe (320), die üblicherweise über eine einen Drucksensor aufweisenden Drucksensorik (330) gesteuert wird. Diese Pumpe (320) ist mit dem ventrikulärem Zustrom (IN) und aortalem Abstrom (OUT) verbunden. Zusätzlich umfasst das Gerät einen Defibrillator bzw. eine Defibrillationseinheit (340) zur Abgabe von Defibrillationsschocks analog einem ICD. Diese Defibrillationseinheit (340) ist mit einer ventrikulären Schockelektrode (HV1) als Defibrillationselektrode verbunden. Diese kann eine separate Schockwendel oder aber integraler Bestandteil der ventrikulären Zustromleitung (IN) sein. Die Gegenelektrode (HV2) wird von einem elektrisch leitenden Gehäuse des implantierbaren kardialen Therapiegerätes (310) gebildet.

Angedeutet ist eine Impedanzmesseinheit (345), die in Verbindung mit den Schockelektroden (HV1 und HV2) einen Impedanzsensor zum Erfassen einer intrakardialen Impedanz.

Die Intrakardiale Impedanz unter anderem vom Blutvolumen im Ventrikel zu einem jeweiligen Zeitpunkt ab, so dass der zeitliche Verlauf der intrakardialen Impedanz die Kontraktionsbewegungen des Ventrikels widerspiegelt. So können aus einem von dem Impedanzsensor stammenden Ausgangssignal in an sich bekannter Weise die Kontraktilität des Ventrikel sowie - im Rahmen der Closed-Loop-Stimulation - den hämodynamischen Bedarf eines Patienten beschreibende Parameter abgeleitet werden, die dann von der Steuereinheit (360) als Therapiesteuereinheit zur Steuerung der Herzunterstützungspumpe (320) und der Defibrillationseinheit (340) ausgewertet werden.

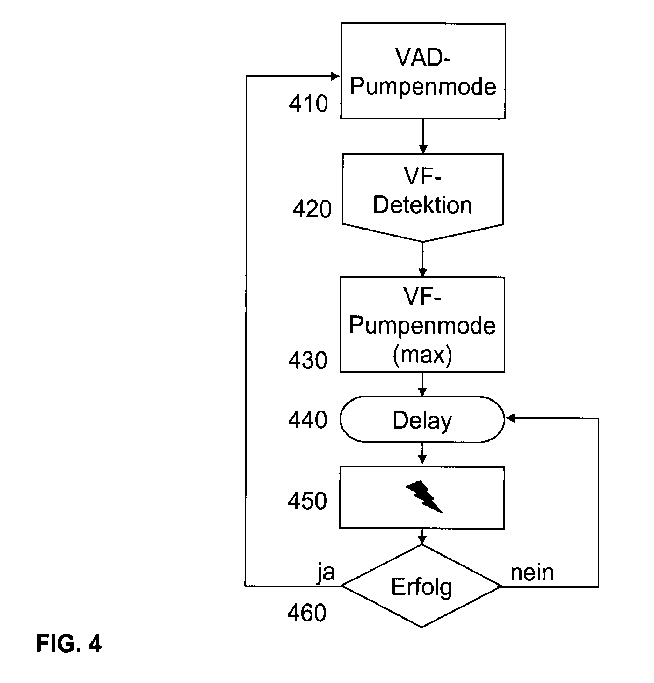

Ferner ist in das Therapiegerät eine Rhythmuswahrnehmungseinheit (350) eingebaut, die den ventrikulären Herzrhythmus üblicherweise über eine bipolare Elektrode (Sense1,2) wahrnimmt und klassifiziert. Die Koordination der kombinierten Therapiefunktionen übernimmt die Therapiesteuereinheit (360), die gemäß Figur 4 die Koordination der Pump- und Defibrillationstherapie übernimmt.

In der Figur 4 ist ein mögliches Ablaufdiagramm für die Steuerung der Kombinationstherapie dargestellt. Das System befindet sich normalerweise im Zustand 410: VAD-Pumpenmode. In diesem Zustand (410) wird ein Teil des benötigten Herzminutenvolumens von der VAD-Pumpe vom linken Ventrikel in die Aorta gepumpt.

Wird nun von der Detektionseinheit ein ventrikuläres Flimmern klassifiziert (420), dann schaltet die Therapiesteuereinheit den Pumpenmode auf eine höhere Leistung, um so den Ventrikel deutlich von der Volumenbelastung des venösen Rückstroms zu entlasten. Nach einer programmierbaren Zeit (440) oder alternativ dem Nachweis geeigneter Druckverhältnisse mittels des VAD eigenen Drucksensors wird nun ein erster Defibrillationsversuch (450) unternommen. Abhängig von Defibrillationserfolg (460) wird anschließend der normale VAD-Pumpenmode (410) aktiviert oder ein weiterer Defibrillationsversuch durch den ggf. modifizierten VF-Pumpenmode vorbereitet.

Die Erfindung bietet den Vorteil, den elektrischen Defibrillationswirkungsgrad und damit die Therapieeffizienz bei Patienten mit stark eingeschränkter Pumpfunktion signifikant zu steigern.

## Patentansprüche

1. Implantierbares kardiales Therapiegerät mit
- einer Herzunterstützungspumpe, die mit einem Ventrikel eines Herzens und einer zugehörigen Arterie zu verbinden und ausgebildet ist, im Einsatzfall Blut aus einem jeweiligen Ventrikel in eine jeweils zugehörige Arterie zu pumpen und so den jeweiligen Ventrikel zu unterstützen/entlasten und
- einer Defibrillationseinheit zur automatischen Defibrillation eines Herzkammerflimmerns, **gekennzeichnet durch**
- eine Steuereinheit, die mit der Herzunterstützungspumpe und der Defibrillationseinheit steuernd verbunden und ausgebildet ist, im Falle eines Herzkammerflimmerns die Herzunterstützungspumpe und die Defibrillationseinheit derart koordiniert anzusteuern, dass die Herzunterstützungspumpe zunächst ihre Leistung steigert um im Einsatzfall zunächst eine Druckentlastung wenigstens eines unterstützen Ventrikels zu bewirken und die Defibrillationseinheit erst anschließend, wenn eine ventrikuläre Druckentlastung vorliegt, einen Defibrillationsschock abgibt.

2. Implantierbares kardiales Therapiegerät kardiales Therapiegerät nach Anspruch 1, **dadurch gekennzeichnet, dass** die Steuereinheit mit einem Drucksensor zum Erfassen eines Druck im jeweiligen Ventrikel verbunden und ausgebildet ist, die Defibrillationseinheit und/oder die Herzunterstützungspumpe in Abhängigkeit eines einen ventrikulären Innendruck anzeigenden Ausgangssignals des Drucksensors zu steuern.

3. Implantierbares kardiales Therapiegerät kardiales Therapiegerät nach Anspruch 1 oder 2, **dadurch gekennzeichnet, dass** die Defibrillationseinheit mit wenigstens einer Defibrillationsslektrode verbunden ist, die zur Abgabe eines Defibrillationsschocks ausgebildet und Bestandteil der Herzunterstützungspumpe oder eines Fluidanschlusses der Herzunterstützungspumpe ist.

4. Implantierbares kardiales Therapiegerät nach einem der Ansprüche 1 bis 3, **dadurch gekennzeichnet, dass** das implantierbare kardiale Therapiegerät einen Sauerstoffsensor aufweist, der mit der Steuereinheit verbunden ist und im Betrieb ein den Blutsauerstoffgehalt anzeigendes Ausgangssignal liefert, wobei die Steuereinheit so ausgebildet ist, dass sie eine Defibrillationsschockabgabe zusätzlich auf Basis des Ausgangssignals des myokardialen Sauerstoffsensors steuert.

5. Implantierbares kardiales Therapiegerät nach einem der Ansprüche 1 bis 4, **dadurch gekennzeichnet, dass** das implantierbare kardiale Therapiegerät einen programmierbaren Zeitgeber aufweist, der mit der Steuereinheit verbunden oder Bestandteil derselben ist, wobei die Steuereinheit so ausgebildet ist, dass sie die Steuerung der Defibrillationsschockabgabe zusätzlich mit Hilfe des programmierbaren Zeitgeber so steuert, dass vor der Defibrillation eine definierte Laufzeit der Unterstützungspumpe mit gesteigerter Leitung sichergestellt ist.

6. Implantierbares kardiales Therapiegerät nach einem der Ansprüche 1 bis 5, **dadurch gekennzeichnet, dass** das implantierbare kardiale Therapiegerät zusätzlich eine Stimulationseinheit zur Post-Schock-Stimulation aufweist, die mit der Steuereinheit verbunden und ausgebildet ist, gesteuert durch die Steuereinheit im Bedarfsfall elektrische Stimulationspulse zur Stimulation einer Herzkammerkontraktion zu liefern.

7. Implantierbares kardiales Therapiegerät nach einem der Ansprüche 1 bis 6, **dadurch gekennzeichnet, dass** das implantierbare kardiale Therapiegerät einen Herzstimulator oder wenigstens eine durch die Steuereinheit gesteuerte Stimulationseinheit aufweist, die ggf. in Kombination mit der Steuereinheit dazu ausgebildet ist, beide Ventrikel eines Herzens im Sinne einer kardialen Resynchronisationstherapie zu stimulieren.

8. Implantierbares kardiales Therapiegerät nach einem der Ansprüche 1 bis 7, **dadurch gekennzeichnet, dass** das implantierbare kardiale Therapiegerät einen Impedanzsensor zum Erfassen einer intrakardialen Impedanz und einen Herzstimulator oder wenigstens eine durch die Steuereinheit gesteuerte Stimulationseinheit aufweist, welche mit der Steuereinheit verbunden ist, wobei die Steuereinheit ausgebildet ist, eine Abgabe von Stimulationsimpulsen in Abhängigkeit eines eine intrakardiale Impedanz anzeigen Ausgangssignals des Impedanzsensors zu steuern.

9. Implantierbares kardiales Therapiegerät nach Anspruch 8, **dadurch gekennzeichnet, dass** die Steuereinheit in Verbindung mit dem Impedanzsensor und der Stimulationseinheit dazu ausgebildet ist, die Stimulation auf Basis eines eine kardiale Kontraktilität anzeigenden Ausgangssignals des Impedanzsensors derart zu steuern, dass eine bedarfsgerechte Steigerung der Kontraktilität resultiert.

10. Implantierbares kardiales Therapiegerät nach einem der Ansprüche 1 bis 9, **dadurch gekennzeichnet, dass** das implantierbare kardiale Therapiegerät Mittel zur Erfassung und Auswertung rhythmologischer Informationen aufweist und die Steuereinheit dazu ausgebildet ist, die Herzunterstützungspumpe auf Basis rhythmologischer Informationen so zu steuern, dass die Herzunterstützungspumpe eine Herz bedarfsgerecht entlastet.

11. Implantierbares kardiales Therapiegerät nach Anspruch 10, **dadurch gekennzeichnet, dass** die Mittel zur Erfassung und Auswertung rhythmologischer Informationen eine oder mehrere Wahrnehmungseinheiten aufweisen, die mit der Steuereinheit verbunden sind, wobei die Mittel zur Erfassung und Auswertung rhythmologischer Informationen ausgebildet sind, ventrikuläre Arrhythmien wie Kammerflimmern und ventrikuläre Tachykardien zu erkennen und zu unterscheiden.

## Claims

1. An implantable cardiac therapy device, comprising
- a cardiac assist pump, which is to be connected to a ventricle of a heart and to an associated artery and which is designed, during use, to pump blood from the respective ventricle into a respective associated artery and to thus assist/relieve the respective ventricle, and
- a defibrillation unit for automatic delivery of a defibrillation shock in response to a ventricular fibrillation, **characterised by**
- a control unit, which is connected to the cardiac assist pump and the defibrillation unit so as to control said pump and unit and which is designed, in the case of ventricular fibrillation, to activate the cardiac assist pump and the defibrillation unit in a coordinated manner in such a way that the cardiac assist pump first increases its performance so as to first relieve the pressure of at least one assisted ventricle during use, and the defibrillation unit only then delivers a defibrillation shock when the pressure of the ventricle is relieved.

2. The implantable cardiac therapy device according to Claim 1, **characterised in that** the control unit is connected to a pressure sensor for detecting a pressure in the respective ventricle and is designed to control a defibrillation unit and/or the cardiac assist pump according to an output signal of the pressure sensor indicating a ventricular inner pressure.

3. The implantable cardiac therapy device according to Claim 1 or 2, **characterised in that** the defibrillation unit is connected to at least one defibrillation electrode, which is designed to deliver a defibrillation shock and is part of the cardiac assist pump or part of a fluid connection of the cardiac assist pump.

4. The implantable cardiac therapy device according to one of Claims 1 to 3, **characterised in that** the implantable cardiac therapy device has an oxygen sensor, which is connected to the control unit and, during operation, supplies an output signal indicating the blood oxygen content, wherein the control unit is designed such that it controls a defibrillation shock delivery additionally on the basis of the output signal of the myocardial oxygen sensor.

5. The implantable cardiac therapy device according to one of Claims 1 to 4, **characterised in that** the implantable cardiac therapy device has a programmable timer, which is connected to the control unit or is part thereof, wherein the control unit is designed such that it controls the control of the defibrillation shock delivery additionally with the aid of the programmable timer, such that a defined runtime of the assist pump with increased performance is ensured before delivery of the defibrillation shock.

6. The implantable cardiac therapy device according to one of Claims 1 to 5, **characterised in that** the implantable cardiac therapy device additionally has a stimulation unit for post-shock stimulation, which is connected to the control unit and is designed to supply electrical stimulation pulses, where necessary, for stimulation of a ventricular contraction in a manner controlled by the control unit.

7. The implantable cardiac therapy device according to one of Claims 1 to 6, **characterised in that** the implantable cardiac therapy device has a cardiac stimulator or at least one stimulation unit, which is controlled by the control unit and which is designed, optionally in combination with the control unit, to stimulate both ventricles of a heart to achieve cardiac resynchronisation therapy.

8. The implantable cardiac therapy device according to one of Claims 1 to 7, **characterised in that** the implantable cardiac therapy device has an impedance sensor for detecting an intracardiac impedance and a cardiac stimulator or at least one stimulation unit, which is controlled by the control unit and which is connected to the control unit, wherein the control unit is designed to control a delivery of stimulation pulses according to an output signal of the impedance sensor indicating an intracardiac impedance.

9. The implantable cardiac therapy device according to Claim 8, **characterised in that** the control unit is designed, in conjunction with the impedance sensor and the stimulation unit, to control the stimulation on the basis of an output signal of the impedance sensor indicating cardiac contractility, in such a way that contractility is increased to meet demand.

10. The implantable cardiac therapy device according to one of Claims 1 to 9, **characterised in that** the implantable cardiac therapy device has means for detecting and evaluating rhythmological information, and the control unit is designed to control the cardiac assist pump on the basis of rhythmological information, such that the cardiac assist pump relieves a heart as required.

11. The implantable cardiac therapy device according to Claim 10, **characterised in that** the means for detecting and evaluating rhythmological information have one or more sensing units, which are connected to the control unit, wherein the means for detecting and evaluating rhythmological information are designed to identify and to distinguish ventricular arrhythmias, such as ventricular fibrillations and ventricular tachycardias.

## Revendications

1. Dispositif thérapeutique cardiaque implantable, avec
- une pompe de soutien cardiaque, destinée à être reliée à un ventricule d'un coeur et à une artère correspondante, et conçue pour pomper du sang à partir d'un certain ventricule vers une certaine artère correspondante, pendant l'utilisation, et pour ainsi soutenir/soulager le ventricule en question, et
- une unité de défibrillateur pour la défibrillation automatique d'une fibrillation ventriculaire, **caractérisé par**
- une unité de commande reliée de façon commandée à la pompe de soutien cardiaque et à l'unité de défibrillateur, et conçue pour actionner la pompe de soutien cardiaque et l'unité de défibrillateur en cas de fibrillation ventriculaire, de manière à ce que la pompe de soutien cardiaque augmente tout d'abord sa puissance, pour provoquer tout d'abord une diminution de pression d'au moins un ventricule soutenu, pendant l'utilisation, et à ce que l'unité de défibrillation n'effectue une défibrillation que par la suite, une fois que la pression ventriculaire a été diminuée.

2. Dispositif thérapeutique cardiaque implantable selon la revendication 1, **caractérisé en ce que** l'unité de commande est reliée à un capteur de pression pour la détection d'une pression dans le ventricule en question, et conçue pour commander l'unité de défibrillation et/ou la pompe de soutien cardiaque en fonction d'un signal de sortie du capteur de pression, indiquant une pression ventriculaire interne.

3. Dispositif thérapeutique cardiaque implantable selon la revendication 1 ou 2, **caractérisé en ce que** l'unité de défibrillation est reliée à au moins une électrode de défibrillation conçue pour émettre un choc de défibrillation et faisant partie de la pompe de soutien cardiaque ou d'un raccord de fluide de la pompe de soutien cardiaque.

4. Dispositif thérapeutique cardiaque implantable selon l'une des revendications 1 à 3, **caractérisé en ce que** le dispositif thérapeutique cardiaque implantable comporte un détecteur d'oxygène relié à l'unité de commande, et fournit un signal de sortie pendant le fonctionnement, indiquant une teneur en oxygène, dans lequel l'unité de commande est conçue de manière à commander une émission de choc de défibrillation en outre sur la base du signal de sortie du capteur d'oxygène myocardiaque.

5. Dispositif thérapeutique cardiaque implantable selon l'une des revendications 1 à 4, **caractérisé en ce que** le dispositif thérapeutique cardiaque implantable comporte un compteur programmable relié à l'unité de commande ou faisant partie de celle-ci, dans lequel l'unité de commande est conçue pour commander l'émission du choc de défibrillation également à l'aide du compteur programmable, de manière à garantir une durée de fonctionnement définie de la pompe de soutien cardiaque avec une puissance augmentée avant la défibrillation.

6. Dispositif thérapeutique cardiaque implantable selon l'une des revendications 1 à 5, **caractérisé en ce que** le dispositif thérapeutique cardiaque implantable comporte en outre une unité de stimulation pour la stimulation postérieure au choc, laquelle est reliée à l'unité de commande et conçue pour délivrer des impulsions de stimulation électriques pour la stimulation d'une contraction ventriculaire, en cas de besoin, tout en étant commandée par l'unité de commande.

7. Dispositif thérapeutique cardiaque implantable selon l'une des revendications 1 à 6, **caractérisé en ce que** le dispositif thérapeutique cardiaque implantable comporte un stimulateur cardiaque ou au moins une unité de stimulation commandée par l'unité de commande, conçue pour stimuler les deux ventricules d'un coeur, au sens d'une thérapie de resynchronisation cardiaque, éventuellement en combinaison avec l'unité de commande.

8. Dispositif thérapeutique cardiaque implantable selon l'une des revendications 1 à 7, **caractérisé en ce que** le dispositif thérapeutique cardiaque implantable comporte un capteur d'impédance pour la détection d'une impédance intracardiaque, ainsi qu'un stimulateur cardiaque ou au moins une unité de stimulation commandée par l'unité de commande et reliée à l'unité de commande, dans lequel l'unité de commande est conçue pour commander une émission d'impulsions de stimulation en fonction d'un signal de sortie du capteur d'impédance, indiquant une impédance intracardiaque.

9. Dispositif thérapeutique cardiaque implantable selon la revendication 8, **caractérisé en ce que** l'unité de commande est conçue pour commander la stimulation sur la base d'un signal de sortie du capteur d'impédance indiquant une contractilité cardiaque, en association avec le capteur d'impédance et l'unité de stimulation, de manière à obtenir une augmentation de la contractilité adaptée aux besoins.

10. Dispositif thérapeutique cardiaque implantable selon l'une des revendications 1 à 9, **caractérisé en ce que** le dispositif thérapeutique cardiaque implantable comporte des moyens pour la détection et l'évaluation d'informations rythmologiques, et l'unité de commande est conçue pour commander la pompe de soutien cardiaque sur la base d'informations rythmologiques, de manière à ce que la pompe de soutien cardiaque soulage un coeur en fonction des besoins.

11. Dispositif thérapeutique cardiaque implantable selon la revendication 10, **caractérisé en ce que** les moyens pour la détection et l'évaluation d'informations rythmologiques comportent une ou plusieurs unités de perception reliées à l'unité de commande, dans lequel les moyens pour la détection et l'évaluation d'informations rythmologiques sont conçue pour reconnaître et distinguer des arythmies ventriculaires, telles qu'une fibrillation ventriculaire ou une tachycardie ventriculaire.
